(19) Europäisches Patentamt
European Patent Office
Office européen des brevets

(11) Veröffentlichungsnummer : **0 361 033 B1**

(12) # EUROPÄISCHE PATENTSCHRIFT

(45) Veröffentlichungstag der Patentschrift : **19.11.92 Patentblatt 92/47**

(51) Int. Cl.$^5$ : **A61K 6/00,** C09J 4/00, C09D 4/00

(21) Anmeldenummer : **89114516.1**

(22) Anmeldetag : **05.08.89**

(54) **Beschichtungsmittel für kollagenhaltige Materialien.**

(30) Priorität : **19.08.88 DE 3828170**

(43) Veröffentlichungstag der Anmeldung : **04.04.90 Patentblatt 90/14**

(45) Bekanntmachung des Hinweises auf die Patenterteilung : **19.11.92 Patentblatt 92/47**

(84) Benannte Vertragsstaaten : **AT BE CH DE ES FR GB GR IT LI LU NL SE**

(56) Entgegenhaltungen :
**EP-A- 0 141 324**
**EP-A- 0 150 952**
**EP-A- 0 321 683**
**WO-A-82/01128**
**PATENT ABSTRACTS OF JAPAN, Band 12, Nr. 96 (C-484)[2943], 29. März 1988**

(73) Patentinhaber : **BAYER AG**
**W-5090 Leverkusen 1 Bayerwerk (DE)**

(72) Erfinder : **Asmussen, Erik, Prof. Dr.**
**Brudevaenget 15**
**Farum (DK)**
Erfinder : **Munksgaard, Erik Christian, Dr.**
**Enebaerhaven 306**
**DK-2980 Kokkedal (DK)**
Erfinder : **Müller, Michael, Dr.**
**Richard-Zanders-Strasse 34**
**W-5060 Bergisch-Gladbach 2 (DE)**
Erfinder : **Podszun, Wolfgang, Dr.**
**Roggendorfstrasse 55**
**W-5000 Köln 80 (DE)**
Erfinder : **Winkel, Jens, Dr.**
**Letterhaus Strasse 1**
**W-5000 Köln (DE)**

EP 0 361 033 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft Zubereitungen, die als Beschichtungsmaterial, z.B. als Grundierungsmaterial (primer oder liner) oder Lack verwendet werden können, um eine Bindung zwischen kollagenhaltigen Materialien und härtenden, polymeren Materialien zu verbessern.

Kollagenhaltige Materialien sind Gerüsteiweißkörper und Hauptbestandteile der menschlichen und tierischen interzellularen Stützsubstanzen wie Knorpel- und Knochengewebe, Haut und Zahnbein (Dentin). Im Rahmen der vorliegenden Erfindung werden die Beschichtungsmittel bevorzugt zur Behandlung von Dentin im Zusammenhang mit Zahnreparaturen verwendet.

Besonders im Dentalbereich werden härtende, polymere Materialien als Füllmaterialien bei Zahnreparaturen verwendet. Als härtende, polymere Materialien werden im allgemeinen Füllungen auf Acrylatbasis bevorzugt. Diese polymeren Füllungen haben jedoch den Nachteil, daß sie schlecht am Dentin haften bleiben. Um dieses Problem zu lösen, hat man bisher teilweise Unterschneidungen am Zahnbein vorgenommen; dazu war es erforderlich, über den angegriffenen Bereich hinaus, beachtliche Mengen an frischem Zahnbein zu entfernen.

Nach einer anderen Methode ätzt man das Zahnbein sowie die Schmelzoberflächen mit Säuren, wie z.B. Phosphorsäure, an und nimmt dann die Füllung vor. Abgesehen davon, daß die Säuren eine Reizwirkung im Mundbereich ausüben, dringen sie auch leicht durch die Dentinkanäle in den Zahn und schädigen den Nerv (pulpa). Außerdem ist bei dieser Methode die Anbindung der Füllung an das Dentin gering.

Aus der EP-A-141 324 sind Beschichtungsmittel für kollagenhaltige Materialien bekannt, die einen Aldehyd, ein olefinisch ungesättigtes Monomer mit aktivem Wasserstoff, gegebenenfalls auch im Gemisch mit einem ungesättigten Monomer ohne aktiven Wasserstoff, Wasser, Lösungsmittel wie Aceton oder Ethanol und übliche Zusätze enthalten (Seite 7, Zeilen 24-29, Seite 10, Zeilen 1-4, und Seite 14, Zeilen 20-22). Ein wesentlicher Gegenstand der der vorliegenden Anmeldung zugrunde liegenden Erfindung ist die Vereinfachung der Vorgehensweise bei einer adhesiven Füllungstherapie, ohne daß ein Festigkeitsverlust der Verklebung auftritt. Das erfindungsgemäße Beschichtungsmaterial ist u.a. auf Zahnbein (Dentin) und Zahnschmelz einsetzbar. Da bei der Versorgung der meisten Kavitäten beide Zahnmaterialien versorgt werden müssen, ist es aus Gründen der Anwendungssicherheit und Zeitsparnis wünschenswert und ein Vorteil, mit dem erfindungsgemäßen Beschichtungsmittel eine Applikationlösung für beide Anforderungen zur Verfügung zu haben. Zu diesem Zweck sind die bereits bekannt gewordenen Adhesive weniger gut geeignet. Zur aufbringung eines Komposites auf Dentin alleine ist nach bisher bekannt gewordenen Verfahren ein zusätzlicher Arbeitsschritt im Vergleich zur vorliegenden Erfindung erforderlich. Dieser zusätzliche Arbeitsschritt umfaßt die Anfertigung einer Zwischenschicht aus einem Verschlußmaterial (EP-A 141 324, Seite 21, Zeile 10-12; "Enamel Binder").

Es hat sich als günstig herausgestellt, wenn man beispielsweise bei Füllungen von Zahnkavitäten zuerst eine Konditionierung der frischen Dentinoberfläche vornimmt. Konditionierungsflüssigkeiten haben im allgemeinen einen pH-Wert von 0,1 bis 3,5 und können eine Säure mit einem $pK_s$-Wert von kleiner als 5 und gegebenenfalls eine amphotere Aminoverbindung mit einem $pK_s$-Wert im Bereich von 9,0 bis 10,6 und einem $pK_B$-Wert im Bereich von 11,5 bis 12,5 enthalten.

Die Beschichtungsmittel sollen zwischen der konditionierten Dentinoberfläche und dem Füllungsmaterial einen festen Verbund ohne Spaltbindung bewirken.

Es wurde ein Beschichtungsmaterial für kollagenhaltige Materialien gefunden, bestehend aus

a) 1-10 Gew.-% Aldehyd,

b) 10-40 Gew.-% wasserlöslichem Monomer mit aktivem Wasserstoff,

c) 1-50 Gew.-% wasserunlöslichem Monomer mit zwei oder mehreren polymerisierbaren Doppelbindungen,

d) 0,01-2,5 Gew.-% Photoinitiator

e) 15-70 Gew.-% Wasser,

f) 0,2-10 Gew.-% Löslichkeitsvermittlern und/oder Dispergatoren

und

g) 0-5 Gew.-% an sich bekannter Zusätze.

Das erfindungsgemäße Beschichtungsmittel bewirkt einen festen Verbund zwischen der konditionierten Dentinoberfläche und dem Füllungsmaterial; die Zahnreparatur bleibt auf diese Weise lange Jahre erhalten. Aldehyde (a) können aliphatische, aromatische oder heterocyclische Aldehyde sein.

Bei aliphatischen Mono- und Dialdehyden ist die Aldehydfunktion an einen aliphatischen, geradlinigen oder verzweigten Kohlenwasserstoffrest mit 1 bis 20, bevorzugt 2 bis 10, insbesondere bevorzugt 2 bis 6, Kohlenstoffatomen gebunden. Als aliphatische Reste seien beispielsweise Methyl, Ethyl, Propyl, Isopropyl, Butyl, Isobutyl, Pentyl, Isopentyl, Hexyl, Isohexyl und Hydroxylethyl genannt. Aliphatische Aldehyde sind beispielsweise Formaldehyd, Acetaldehyd, Butyraldehyd, Glutardialdehyd und Glyoxal.

Bei aromatischen Aldehyden ist die Aldehydfunktion an einen aromatischen Rest gebunden. Als aromatische Reste seien aromatische Kohlenwasserstoffe mit 6 bis 12 Kohlenstoffatomen, vorzugsweise Phenyl, Naphthyl und Biphenyl, genannt. Die aromatischen Reste können beispielsweise substituiert sein durch Alkyl($C_1$ bis $C_6$), Alkoxyl ($C_1$ bis $C_6$), Hydroxyl oder Carboxyl.

Als aromatische Aldehyde seien beispielsweise genannt: Benzaldehyd, Salicylaldehyd, Vanillin, o-Phthaladehyd.

Bei heterocyclischen Aldehyden ist die Aldehydfunktion an den Heterocyclus gebunden. Beispielhaft sei Furfurylaldehyd genannt.

Die Aldehyde für die erfindungsgemäßen Beschichtungsmittel können Mono- oder Dialdehyde sein.

Für die erfindungsgemäßen Beschichtungsmittel werden aliphatische Mono- und Dialdehyde bevorzugt.

Als wasserlösliche Monomere sind ungesättigte Monomere zu verstehen, die eine Wasserlöslichkeit bei 20°C von über 5 Gew.-%, bevorzugt 10 bis 100 Gew.-%, bezogen auf die Mischung aus Monomer und Wasser aufweisen.

Bevorzugt werden Acrylsäure und Methacrylsäure und deren Derivate, wie die Säureamide und Hydroxyalkylester-($C_2$ bis $C_6$).

Beispielsweise sei (Meth)acrylsäure, Methacrylsäureamid, Hydroxyalkyl($C_2$ bis $C_5$)methacrylate wie Hydroxyethyl-methacrylat und Hydroxypropylmethacrylat, sowie Ethylen-, Diethylen- und Triethylenglykolmonomethacrylat genannt.

Die wasserunlöslichen, ungesättigten Monomere besitzen eine Wasserlöslichkeit bei 20°C von unter 5 Gew.-%, bevorzugt 0,01 bis 3 Gew.-%. Sie enthalten zwei oder mehrere polymerisierbare Doppelbindungen im Molekül, wobei besonders Methacryl und Acrylsäureester von 2- bis 5-wertigen Alkoholen mit 2 bis 30 Kohlenstoffatomen in Betracht kommen. Besonders bevorzugt sind Alkoxy-(meth)acrylate und Urethangruppen enthaltende (Meth)acrylate.

Beispielsweise seien (Meth)acrylsäureester der Formel

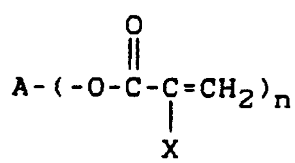

in der

A einen n-wertigen, geradkettigen, verzweigten, cyclischen, aliphatischen, aromatischen oder gemischt aliphatisch-aromatischen Rest mit 2 bis 25 C-Atomen, der durch -O- oder NH-Brücken unterbrochen und mit Hydroxy, Oxy, Carboxy, Amino oder Halogen substituiert sein kann,

bedeutet,

X H oder Methyl bedeutet und

n für eine ganze Zahl von 2 bis 8, vorzugsweise 2 bis 4, steht,

genannt.

Vorzugsweise seien Verbindungen der folgenden Formeln genannt:

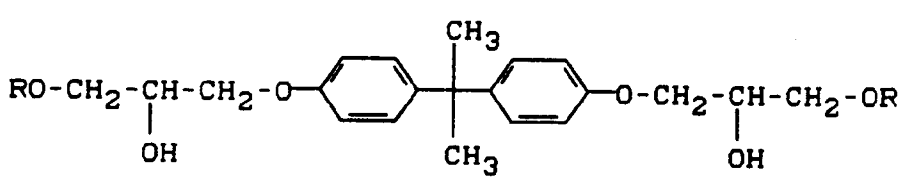

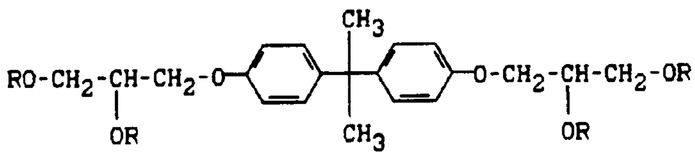

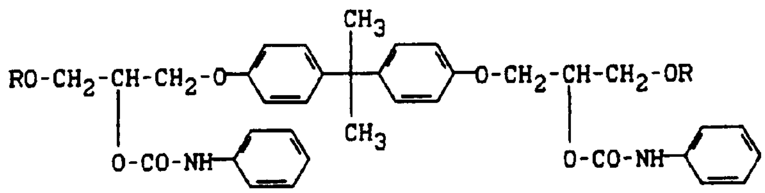

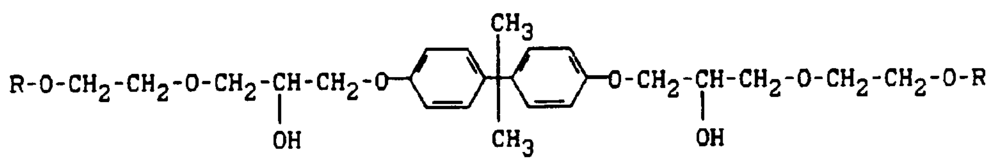

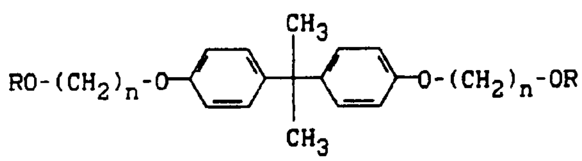

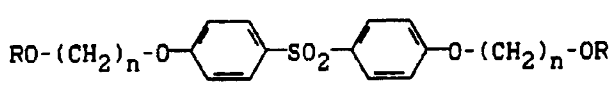

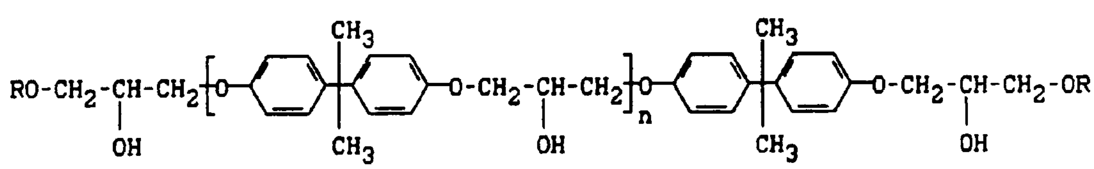

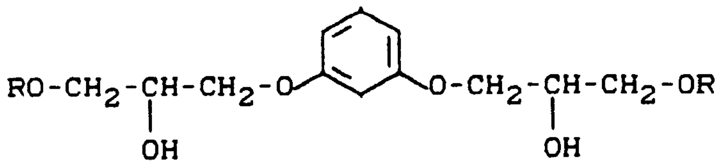

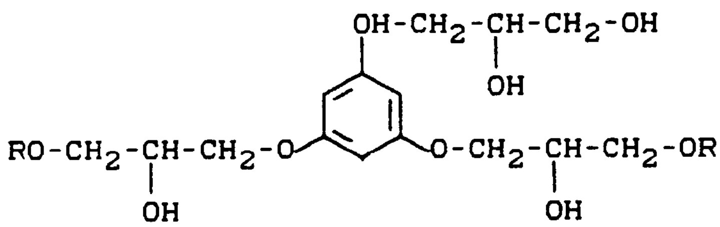

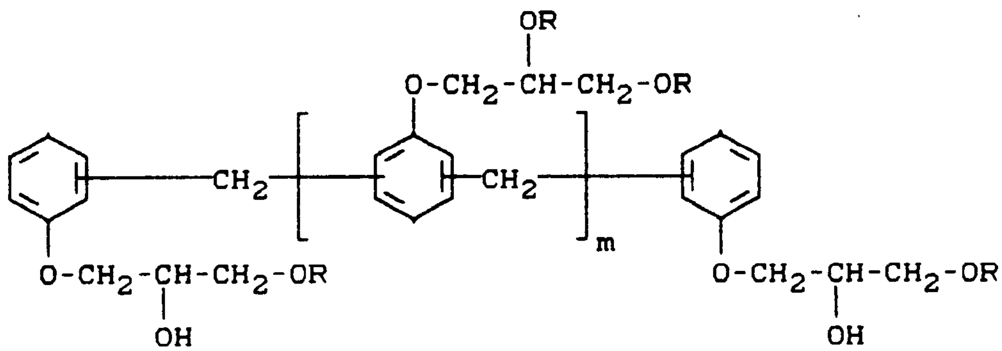

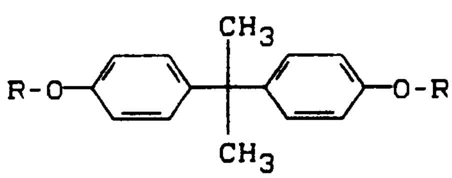

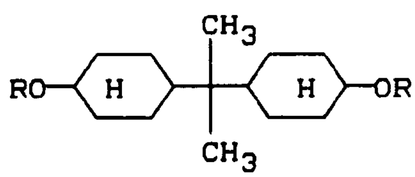

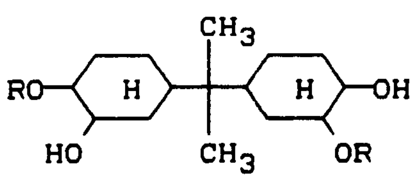

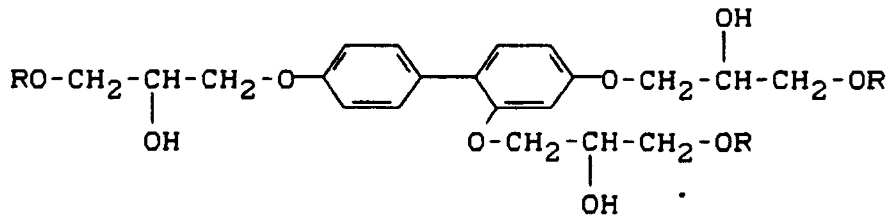

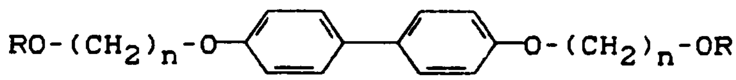

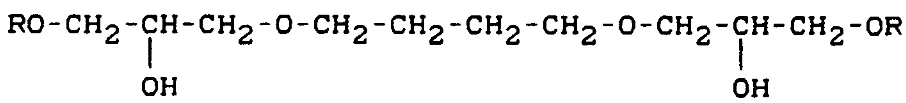

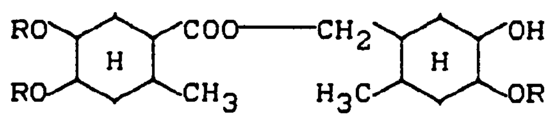

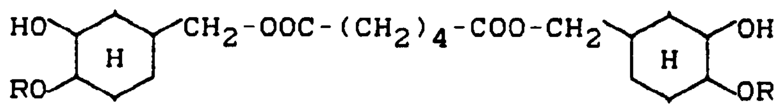

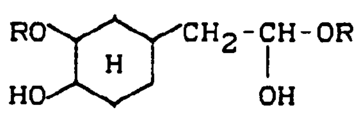 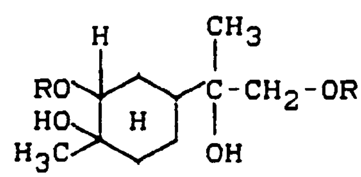

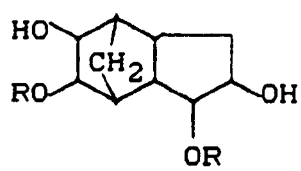

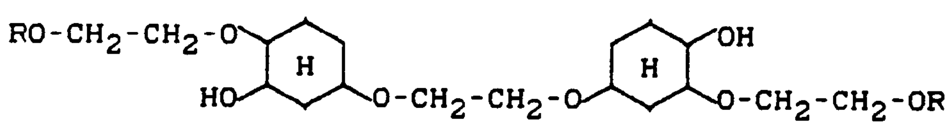

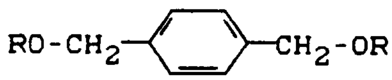

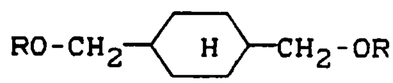

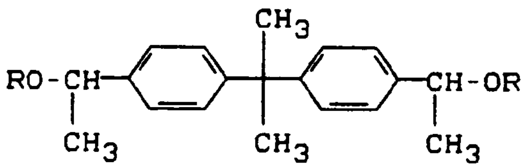

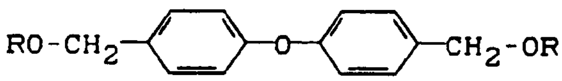

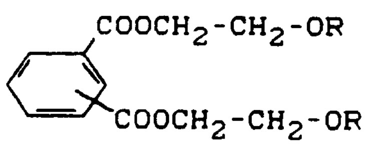

in der ortho-, meta- oder para-Form

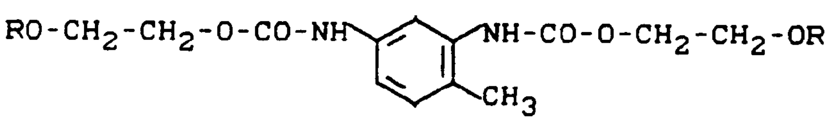

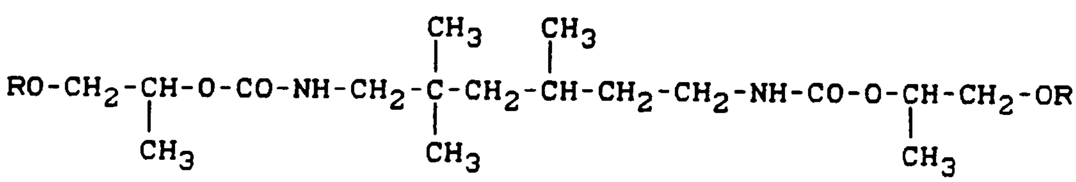

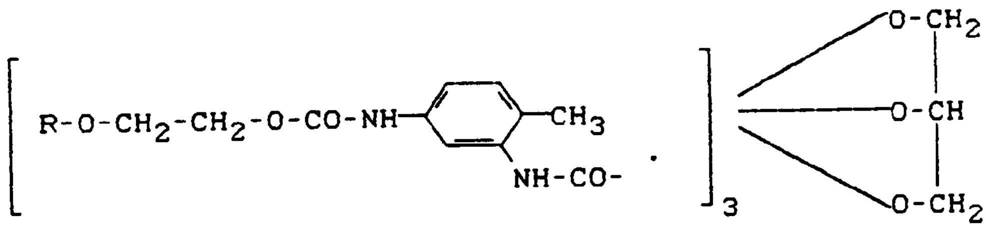

$R-O-(CH_2)_n-O-R$

$R-O-(CH_2CH_2O)_m-R$

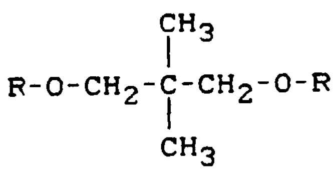

worin
R für

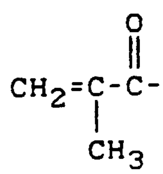

oder

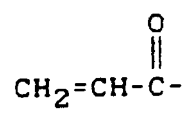

steht,

n eine Zahl von 1 bis 4 und
m eine Zahl von 0 bis 5 bedeutet.

Außerdem seien Derivate des Tricyclodecans (EP-A 00 23 686) und Umsetzungsprodukte aus Polyolen, Diisocyanaten und Hydroxyalkylmethacrylaten (DE-A 37 03 120, DE-A 37 03 080 und DE-A 37 03 130) genannt.

Besonders bevorzugt als Monomer wird das sogenannte Bis-GMA der Formel

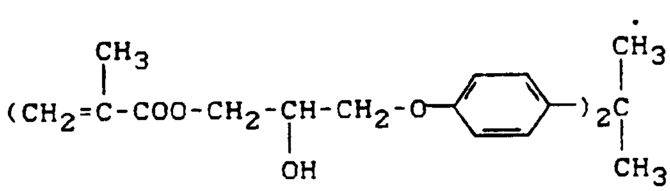

Selbstverständlich ist es möglich, Mischungen der verschiedenen (Meth)acrylsäureester, die Vernetzungen ausbilden können, einzusetzen. Beispielsweise seien Mischungen von 20 bis 70 Gew.-Teilen Bis-GMA und 30 bis 80 Gew.-Teilen Triethylenglykoldimethacrylat genannt.

Photoinitiatoren (d) sind an sich bekannt (Houben-Weyl, Methoden der organischen Chemie, Band E20, S. 80 ff, Georg Thieme Verlag Stuttgart. 1987). Vorzugsweise handelt es sich dabei um Carbonylverbindungen, wie Benzoin und dessen Derivate, insbesondere Benzoinmethylether, Benzil und Benzilderivate, beispielsweise 4,4-Oxidibenzil und andere Dicarbonylverbindungen wie Diacetyl, 2,3-Pentandion und α-Diketo-Derivate des Norbornans und substituierter Norbornane wie Campherchinon, Metallcarbonyle wie Pentacarbonylmangan, Chinone wie 9,10-Phenanthrenchinon und Naphthochinon.

Die erfindungsgemäßen Beschichtungsmaterialien enthalten vorzugsweise noch Coaktivatoren, die in Gegenwart von Fotopolymerisationsinitiatoren die Polymerisationsreaktion beschleunigen. Bekannte Beschleuniger sind beispielsweise aromatische Amine wie p-Toluidin und Dimethyl-p-toluidin, Trialkylamine wie Trihexylamin, Polyamine wie N,N,N',N'-Tetraalkylalkylendiamine, Barbitursäure und Dialkylbarbitursäure.

Die Beschleuniger sind im allgemeinen in einer Menge von 0,01 bis ca. 5 Gew.-% im Beschichtungsmaterial enthalten.

Als Coaktivatoren seien auch Alkylaminoarylsulfonamide der Formel

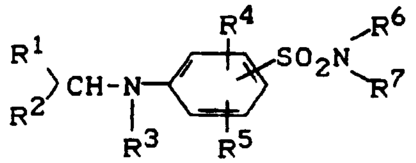

,

worin

$R^1$ und $R^2$ gleich oder verschieden sind und Wasserstoff, Methyl, Vinyl oder Phenyl bedeuten,

$R^3$ Wasserstoff, Methyl und Ethyl bedeutet,

$R^4$ und $R^5$ gleich oder verschieden sind und Wasserstoff, Halogen oder Methyl bedeuten oder orthoständiges $R^4$ und $R^5$ Teile eines anellierten aromatischen Sechsrings sein können,

$R^6$ und $R^7$ gleich oder verschieden sind und Wasserstoff, Methyl, Allyl, Methallyl, Cyclohexyl, Phenyl, Benzyl, Hydroxyethyl, Hydroxypropyl, Acryloyloxyalkyl, Methacryloyloxyalkyl, 2,3-Epoxypropyl oder 1,2-Dihydroxypropyl-1-(meth)acrylat bedeuten und wobei $R^6$ und $R^7$ zu einem Morpholin- oder Piperidinrest verbunden sein können,

genannt.

Als Alkylaminoarylsulfonamide seien beispielsweise genannt:

N,N-Dimethyl-p-dimethylaminobenzolsulfonsäureamid und N-Hydroxyethyl-p-dimethylaminobenzolsulfonsäureamid.

Wasser (e) kann beispielsweise demineralisiertes Wasser sein.

Löslichkeitsvermittler im Rahmen der vorliegenden Erfindung sind flüchtige Lösungsmittel (Dampfdruck bei 25°C über 1000 Pa) aus der Gruppe der Ketone, Alkohole und Ether. Bevorzugt werden aliphatische Ketone mit 3 bis 6 Kohlenstoffatomen, aliphatische Alkohole mit 1 bis 5 Kohlenstoffatomen, Glykolmonoalkyl($C_1$ bis $C_4$)ether und cyclische Ether($C_4$ bis $C_6$). Beispielhaft seien genannt: Aceton, Butanon, Methanol, Ethanol, Propanol, Isopropanol, Butanol, Isobutanol, sek.-Butanol, tert.-Butanol, Ethylenglykolmonomethylether, Tetrahydrofuran und 1,4-Dioxan.

Erfindungsgemäß einsetzbare Dispergatoren (f) können ionische und nichtionische Tenside sein.

Bevorzugte anionische Tenside sind beispielsweise: Phosphate wie Di-(2-ethylhexyl)-phosphat-Na-Salz, Alkoholsulfate wie Oleylalkoholsulfat, Alkyl($C_6$ bis $C_{20}$)benzolsulfonate und Alkyl($C_8$ bis $C_{24}$)sulfonate, insbesondere Sulfobernsteinsäuredioctylester-Na-Salz.

Bevorzugte kationische Tenside können beispielsweise sein: quartäre Ammoniumsalze wie beispielsweise Methyltrioctylammoniumchlorid und Methyl-tricaprylylammoniumchlorid.

Erfindungsgemäß einsetzbar sind auch nichtionogene Tenside in Form von Fettsäurederivaten von Polyolen oder des Ethylenoxids, ethoxylierte Fettalkohole und Phenole sowie amphotere Tenside wie Alkylaminoethansulfonsäure. Besonders bevorzugt sind oberflächenaktive, hochmolekulare Verbindung. Genannt seien hier wasserlösliche Polyvinylverbindungen wie Polyvinylacetat, Polymethacrylsäure und Polyacrylsäure sowie deren Alkalisalze, Copolymerisate aus Natriummethacrylat und Methacrylsäurealkylester. Weiterhin gut geeignet sind Cellulosederivate wie Methylcellulose und Carboxymethylcellulose. Besonders gut geeignet ist Poly-N-vinylpyrrolidon (2).

An sich bekannte Zusätze können Stabilisatoren, Inhibitoren, Lichtschutzmittel, Farbstoffe, Pigmente und Fluoreszenzstoffe sein.

Bevorzugt werden Beschichtungsmittel für kollagenhaltige Materialien, die aus

a) 2 bis 8 Gew.-% Aldehyd,

b) 15 bis 40 Gew.-% wasserlöslichem Monomer mit aktivem Wasserstoff,

c) 2 bis 40 Gew.-% wasserunlöslichem Monomer mit zwei oder mehreren polymerisier baren Doppelbindungen,

d) 0,01 bis 2,5 Gew.-% Photoinitiator,

e) 25 bis 60 Gew.-% Wasser,

f) 0,5 bis 10 Gew.-% Löslichkeitsvermittler und/oder Dispergatoren

g) 0 bis 5 Gew.-% an sich bekannter Zusätze bestehen.

Vor der Beschichtung eines kollagenhaltigen Materials mit dem erfindungsgemäßen Beschichtungsmaterial wird dieses im allgemeinen mit einer Konditionierflüssigkeit behandelt. Die Zusammensetzung der Konditionierflüssigkeit richtet sich nach der Art des zu beschichtenden Materials. Für Zahnschmelz eignen sich besonders gut 15-60 %-ige wäßrige Lösungen von Orthophosphorsäure. Als Konditionierlösungen für Zahnbein (Dentin) können beispielsweise wäßrige Lösungen von EDTA (z.B. 0,5 molar, mit NaOH auf pH 7,4 eingestellt) oder wäßrige Lösungen, die 10 % Zitronensäure und 3 %- Eisen(III)chlorid enthalten, eingesetzt werden.

Konditionierflüssigkeiten für ein breites Einsatzgebiet, die insbesondere für Zahnschmelz und Zahnbein einsetzbar sind, enthalten Säuren mit einem $pK_s$-Wert kleiner als 5 und gegebenenfalls eine amphotere Aminoverbindung mit einem $pK_s$-Wert im Bereich von 9,0 bis 10,6 und einem $pK_B$-Wert im Bereich von 11,5 bis 12,5. Folgende Säuren können in den Konditionierflüssigkeiten enthalten sein: Phosphorsäure, Salpetersäure, Brenztratubensäure, Zitronensäure, Oxalsäure, Ethylendiamintetraessigsäure, Essigsäure, Weinsäure, Äpfelsäure, Maleinsäure.

Als amphotere Aminoverbindungen seien vorzugsweise Verbindungen der Formel

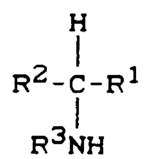

,

in der

$R^1$ für eine Carboxylgruppe steht,

$R^2$ Wasserstoff, gegebenenfalls durch Hydroxy, Thio, Methylthio, Carboxy, Amino, Phenyl, Hydroxyphenyl oder die Gruppen

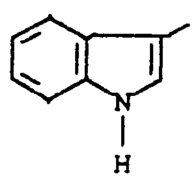

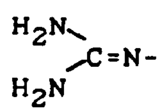

,

substituierter Niederalkylrest,

$R^3$ Wasserstoff oder Phenyl bedeutet und

wobei die Reste $R^1$ und $R^3$ durch einen Propylrest verbunden sein können, oder

in der

$R^1$ für Wasserstoff steht,

$R^2$ die Gruppe $-A-NH_3X$,

in der

A für einen zweibindigen Alkylenrest mit 1 bis 6 Kohlenstoffatomen und

X für Halogen steht,

bedeutet und

$R^3$ Wasserstoff bedeutet,

genannt.

Beispielsweise seien die folgenden amphoteren Aminoverbindungen genannt: Glycin, Serin, Threonin, Cystein, Thyrosin, Asparagin, Glutamin, Alanin, Valin, Leucin, Isoleucin, Prolin, Methionin, Phenylalanin,

Tryptophan, Lysin, Arginin, Histidin, N-Phenylglycin, Ethylendiaminhydrochlorid, Propylendiaminhydrobromid, Butylendiaminhydrochlorid, Butylendiaminhydrobromid, Leucinhydrochlorid und Histidinhydrochlorid.

Weiterhin kann die Konditionierflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden, solange freie Säurefunktionen verbleiben.

Konditionierflüssigkeiten, die mindestens eine der Säuren aus der Gruppe der Brenztraubensäure, Ethylendiamintetraessigsäure und Zitronensäure sowie gegebenenfalls eine amphotere Aminoverbindung aus der Gruppe des Glycins, N-Phenylglycins und Prolins enthalten, sind besonders bevorzugt.

Die erfindungsgemäßen Beschichtungsmittel stellt man im allgemeinen durch Mischen der Komponenten her.

Die erfindungsgemäßen Zubereitungen können als Beschichtungsmittel, vorzugsweise als Grundierungsmittel (primer oder liner) oder Lack (varnish) verwendet werden, um die Bindung zwischen kollagenhaltigen Materialien und härtenden, polymeren Materialien zu verbessern.

Kollagenhaltige Materialien kommen an vielen Stellen des menschlichen und tierischen Körpers vor. Die erfindungsgemäße Anwendung betrifft selbstverständlich lebende und nicht lebende Materialien. Als kollagenhaltige Materialien seien Zähne, Knochen, Haut und Leder genannt. Bevorzugt werden die erfindungsgemäßen Zubereitungen zur Beschichtung von Zähnen zur Vorbereitung von Zahnfüllungen bei Zahnreparaturen verwendet.

Die härtenden polymeren Materialien werden im wesentlichen durch das Anwendungsgebiet bestimmt. So können beispielsweise im Dentalbereich für die Polymerisation nur Monomere eingesetzt werden, die physiologisch unbedenklich sind und die im Mundbereich polymerisieren können, beispielsweise sei Bis-GMA (Bisphenol-A-diglycidyldimethacrylat) genannt.

Bei der Anwendung, z.B. bei einer Zahnreparatur trägt man nach mechanischer Reinigung des kollagenhaltigen Zahnmaterials zuerst die beschriebene Konditionierflüssigkeit mit etwas Watte auf, läßt sie 60 Sekunden einwirken, spült das Zahnmaterial mit Wasser und trocknet es im Luftstrom. Das erfindungsgemäße Beschichtungsmaterial wird anschließend mit einem kleinen Pinsel in dünner Schicht aufgetragen und überschüssiges Material im Luftstrom verteilt. Auf das Beschichtungsmaterial wird nun die Zahnfüllmasse aufgetragen und mit diesem zusammen ausgehärtet.

<u>Beispiele 1 bis 5</u>

Erfindungsgemäße Beschichtungsmaterialien

Mit Hilfe eines hochtourigen Rührers (2000 UpM) werden Mischungen mit den unten aufgeführten Zusammensetzungen hergestellt.

<u>Beispiel 1</u>

| | |
|---|---|
| 5 g | Glutardialdehyd |
| 33 g | 2-Hydroxyethylmethacrylat |
| 2 g | Bis-GMA |
| 0,1 g | Campherchinon (Fotoinitiator) |
| 55 g | Wasser |
| 5 g | Aceton |

<u>Beispiel 2</u>

| | |
|---|---|
| 5 g | Glutardialdehyd |
| 33 g | 2-Hydroxyethylmethacrylat |
| 2 g | Bis-GMA |
| 0,1 g | Campherchinon |
| 59,2 g | Wasser |
| 0,7 g | Poly-N-vinyl-2-pyrrolidon |

<u>Beispiel 3</u>

| | |
|---|---|
| 3,3 g | Glutardialdehyd |
| 23,4 g | 2-Hydroxyethylmethacrylat |

| | |
|---|---|
| 12,6 g | Triethylenglykoldimethacrylat |
| 20,5 g | Bis-GMA |
| 67 mg | Campherchinon |
| 167 mg | N,N-Dimethylaminobenzolsulfonsäurediallylamid (Coaktivator) |
| 38,1 g | Wasser |
| 2 g | Poly-N-vinyl-2-pyrrolidon |
| 33 mg | 2,6-Di-tert.-butyl-4-methylphenol (Stabilisator) |

Beispiel 4

| | |
|---|---|
| 3,3 g | Glutardialdehyd |
| 23,4 g | 2-Hydroxyethylmethacrylat |
| 20,8 g | Bis-GMA |
| 12,7 g | Triethylenglykoldimethacrylat |
| 70 mg | Campherchinon |
| 39,3 g | Wasser |
| 0,5 g | Methyl-tricaprylammoniumchlorid |

Beispiel 5

| | |
|---|---|
| 2,5 g | Glutardialdehyd |
| 18 g | 2-Hydroxyethylmethacrylat |
| 30 g | Bis-GMA |
| 17,5 g | Triethylenglykoldimethacrylat |
| 100 mg | Campherchinon |
| 29,5 g | Wasser |
| 2,5 g | Poly-N-vinyl-2-pyrrolidon |

Beispiel 6

Anwendungstechnische Prüfung

Die Eignung der Beschichtungsmaterialien der Beispiele 1 bis 5 wird geprüft, indem die Bindungsfestigkeit eines Kunststoffmaterials, das Bis-GMA und Triethylenglykoldimethacrylat als Monomere und Campherchinon als Fotoinitiator enthält, auf Dentin und Schmelz bestimmt wird, die mit einer Konditionierflüssigkeit (60 Sekunden Einwirkzeit, Wasserspülung, Lufttrocknung) und dem Beschichtungsmaterial (60 Sekunden Einwirkzeit, Lufttrocknung) vorbehandelt worden sind.

Für den Test werden herausgezogene und im feuchten Zustand aufbewahrte Menschenzähne benutzt. Die Zähne werden durch Guß in Epoxidharz eingelagert. Für die Messung auf Dentin wird durch Naßschleifen eine glatte Dentinoberfläche erzeugt. Das abschließende Schleifen erfolgt mit Carbonpapier 1000.

Zur Herstellung eines Probekörpers zum Messen der Bindungsstärke wird eine zylindrische, gespaltete Teflonform auf die wie vorstehend beschrieben behandelte Dentinoberfläche gespannt. (Scand. J. Dent. Res. 88, 348-351 (1980)). Als Füllmasse wird das fotohärtende Kunststoff-Füllungsmaterial eingefüllt. Ein in einem Loch in einer Bohrerhaltung eingespannter Rundbohrer Nr. 016 wird an der Teflonform befestigt und von oben in die noch im Härteprozeß befindliche Materialschicht gepreßt.

Die gesamte Anordnung wird 10 Min. lang bei Zimmertemperatur (23 ± 2°C) ungestört stehengelassen, wonach die Bohrerhalterung und die Teflonform abgenommen und die Probe unter Wasser mit einer Temperatur von 23 ± 1°C abgesetzt wird. Nach 24 Stdn. Wird die Probe mit dem Bohrer in eine Instron-Zugversuchsapparatur (Scand. J. Dent. Res. 88, 348-351 (1980)) montiert; mit einer Geschwindigkeit von 1 mm/Min. Wird eine Zugfestigkeitsmessung durchgeführt. Die Zugfestigkeit errechnet sich aus der Division der beim Bruch der Füllung angelegten Belastung mit dem Querschnittsareal in der Bruchfläche des Probekörpers. Es wurden jeweils 5 Messungen an Probekörpern durchgeführt. Als Konditionsflüssigkeiten wurden dabei die folgenden Lösungen verwendet:

K 1:

9,1 % Brenztraubensäure
9,1 % Glycin
81,8 % Wasser

K 2:

17 % Ethylendiamintetraessigsäure-dinatriumsalzdihydrat
1,7 % Natriumhydroxid
81,3 % Wasser

K 3:

35 % Orthophosphorsäure
65 % Wasser.

Die Ergebnisse der Bindungsfestigkeitsmessungen sind in der folgenden Tabelle zusammengefaßt:

| Beschichtungsmaterial | Dentin Konditionierflüssigkeit | Dentin Bindungsfestigkeit $[N/mm^2]$ | Schmelz Konditionierflüssigkeit | Schmelz Bindungsfestigkeit $[N/mm^2]$ |
|---|---|---|---|---|
| Beispiel 1 | K 1 | 15 ± 4 | K 1 | 14,8 ± 0,5 |
| Beispiel 2 | K 1 | 19 ± 2 | K 1 | 16,2 ± 3,7 |
| Beispiel [illegible] | K 2 | 12,3 ± 1 | | |
| Beispiel 3 | K 2 | 11,8 ± 4 | K 3 | 12,6 ± 3,2 |
| Beispiel 4 | K 2 | 13,5 ± 3 | K 3 | 17,0 ± 2,1 |
| Beispiel 5 | K 2 | 14,1 ± 3 | K 3 | 13,2 ± 3,5 |

Beispiel 7 (Vergleichsversuch)

Es wurde ein Beschichtungsmaterial der folgenden Zusammensetzung hergestellt:

2,5 g Glutardialdehyd

| | |
|---|---|
| 18 g | 2-Hydroxyethylmethacrylat |
| 30 g | Bis-GMA |
| 17,5 g | Triethylenglykoldimethacrylat |
| 100 mg | Campherchinon |
| 7,5 g | Wasser |
| 24,5 g | Aceton. |

Nach der in Beispiel 6 beschriebenen Arbeitsweise wurden Bindungsfestigkeiten auf Dentin und Schmelz ermittelt.

| Dentin Konditionier-flüssigkeit | Bindungs-festigkeit [N/mm²] | Schmelz Konditionier-flüssigkeit | Bindungs-festigkeit [N/mm²] |
|---|---|---|---|
| K 2 | 1,0 | K 3 | 7,2 |

## Patentansprüche

### Patentansprüche für folgend Vertragsstaaten: AT,BE,CH,DE,FR,GB,GR, IT,LI,LU,NL,SE

1. Beschichtungsmittel für kollagenhaltige Materialen, bestehend aus
   a) 1-10 Gew.-% Aldehyd,
   b) 10-40 Gew.% wasserlöslichem Monomer mit aktivem Wasserstoff,
   c) 1-50 Gew.-% wasserunlöslichem Monomer mit zwei oder mehreren polymerisierbaren Doppelbindungen
   d) 0,01-2,5 Gew.-% Photoinitiator,
   e) 15-70 Gew.-% Wasser,
   f) 0,2-10 Gew.-% Löslichkeitsvermittlern und/oder Dispergatoren,
   und
   g) 0-5 Gew.-% an sich bekannter Zusätze.

2. Beschichtungsmittel nach Anspruch 1,
   bestehend aus
   a) 2 bis 8 Gew.-% Aldehyd,
   b) 15 bis 40 Gew.-% wasserlöslichem Monomer mit aktivem Wasserstoff,
   c) 2 bis 40 Gew.-% wasserunlöslichem Monomer mit zwei oder mehreren polymerisierbaren Doppelbindungen.
   d) 0,01 bis 2,5 Gew.-% Photoinitiator
   e) 25 bis 60 Gew.-% Wasser,
   f) 0,5 bis 10 Gew.-% Löslichkeitsvermittlern und/oder Dispergatoren
   und
   g) 0 bis 5 Gew.-% an sich bekannter Zusätze.

3. Beschichtungsmaterial nach den Ansprüchen 1 bis 2,
   dadurch gekennzeichnet, daß als Komponente f) ein Dispergator eingesetzt wird.

4. Beschichtungsmaterial nach den Ansprüchen 1 bis 3,
   dadurch gekennzeichnet, daß als Dispergator Poly-N-vinyl-2-pyrrolidon eingesetzt wird.

5. Verfahren zur Herstellung von Beschichtungsmaterial für kollagenhaltiges Material, dadurch gekennzeichnat, daß man

a) 1-10 Gew.-% Aldehyd,
b) 10-40 Gew.-% wasserlösliches Monomer mit aktivem Wasserstoff,
c) 1-50 Gew.-% wasserunlösliches Monomer mit zwei oder mehreren polymerisierbaren Doppelbindungen,
d) 0,01-2,5 Gew.-% Photoinitiator,
e) 15-70 Gew.-% Wasser,
f) 0,2-10 Gew.-% Löslichkeitsvermittler und/oder Dispergatoren,
und gegebenenfalls
g) 0-5 Gew.-% an sich bekannte Zusätze,
unter Rühren mischt.

**Patentanspruch für folgenden Vertragstaat: ES**

1. Verfahren zur Herstellung von Beschichtungsmaterial für kollagenhaltiges Material, dadurch gekennzeichnat, daß man
a) 1-10 Gew.-% Aldehyd,
b) 10-40 Gew.-% wasserlösliches Monomer mit aktivem Wasserstoff,
c) 1-50 Gew.-% wasserunlösliches Monomer mit zwei oder mehreren polymerisierbaren Doppelbindungen,
d) 0,01-2,5 Gew.-% Photoinitiator,
e) 15-70 Gew.-% Wasser,
f) 0,2-10 Gew.-% Löslichkeitsvermittler und/oder Dispergatoren,
und gegebenenfalls
0-5 Gew.-% an sich bekannte Zusätze,
unter Rühren mischt.

## Claims

**Claims for the following Contracting States: AT,BE,CH,DE,FR,GB,GR, IT,LI,LV,NL,SE**

1. Coating agent for collagen-containing materials, consisting of
a) 1-10 % by weight of an aldehyde,
b) 10-40 % by weight of a water-soluble monomer having active hydrogen,
c) 1-50 % by weight of a water-insoluble monomer having two or more polymerizable double bonds,
d) 0.01-2.5 % by weight of photoinitiator,
e) 15-70 % by weight of water,
f) 0.2-10 % by weight of solubilizers and/or dispersants and
g) 0-5 % by weight of additives known per se.

2. Coating agent according to Claim 1, consisting of
a) 2 to 8 % by weight of an aldehyde,
b) 15 to 40 % by weight of a water-soluble monomer having active hydrogen,
c) 2 to 40 % by weight of a water-insoluble monomer having two or more polymerizable double bonds,
d) 0.01 to 2.5 % by weight of photoinitiator,
e) 25 to 60 % by weight of water,
f) 0.5 to 10 % by weight of solubilizers and/or dispersants and
g) 0 to 5 % by weight of additives know per se.

3. Coating material according to Claims 1 to 2,
characterized in that a dispersant is used as the component f).

4. Coating material according to Claims 1 to 3,
characterized in that poly-N-vinyl-2-pyrrolidone is used as the dispersant.

5. Process for preparing coating material for collagen-containing material, characterized in that
a) 1-10 % by weight of an aldehyde,
b) 10-40 % by weight of a water-soluble monomer having active hydrogen,

c) 1-50 % by weight of a water-insoluble monomer having two or more polymerizable double bonds,
d) 0.01-2.5 % by weight of photoinitiator,
e) 15-70 % by weight of water,
f) 0.2-10% by weight of solubilizers and/or dispersants and optionally
g) 0-5 % by weight of additives known per se, are mixed with stirring.

**Claim for the following Contracting State: ES**

1. Process for preparing coating material for collagen-containing material, characterized in that
   a) 1-10 % by weight of an aldehyde,
   b) 10-40 % by weight of a water-soluble monomer having active hydrogen,
   c) 1-50 % by weight of a water-insoluble monomer having two or more polymerizable double bonds,
   d) 0.01-2.5 % by weight of photoinitiator,
   e) 15-70 % by weight of water,
   f) 0.2-10% by weight of solubilizers and/or dispersants and
   g) 0-5 % by weight of additives known per se, are mixed with stirring.

## Revendications

**Revendications pour les Etats contractants suivants : AT,BE,GH,DE,FR, GB,GR,IT,LI,LU,NL,SE**

1. Agent d'enduction pour matériaux contenant du collagène constitué de :
   a) 1 - 10 % en poids d'aldéhyde,
   b) 10 - 40 % en poids de monomère soluble dans l'eau avec de l'hydrogène actif,
   c) 1 à 50 % en poids de monomère insoluble dans l'eau avec deux ou plusieurs liaisons doubles polymérisables,
   d) 0,01 - 2,5% en poids de photo-initiateur,
   e) 15 à 70 % en poids d'eau,
   f) 0,2 à 10 % en poids de solubilisant et/ou de dispersant, et
   g) 0 à 5% en poids d'additif connu en soi.

2. Agent d'enduction selon la revendilcation 1, constitué de :
   a) 2 à 8% en poids d'aldéhyde,
   b) 15 à 40% en poids de monomère soluble dans l'eau avec hydrogène actif,
   c) 2 à 40% en poids de monomère insoluble dans l'eau avec deux ou plusieurs liaisons doubles polymérisables,
   d) 0,01 à 2,5% en poids de photo-initiateur
   e) 25 à 60% en poids d'eau,
   f) 0,5 à 10% en poids de solubilisant et/ou de dispersant et éventuellement
   g) 0 à 5% en poids d'additif connu en soi.

3. Matériau d'enduction selon les revendications 1 à 2, caractérisé en ce que l'on utilise comme constituant f) un agent de dispersion.

4. Matériau d'enduction selon les revendications 1 à 3, caractérisé en ce que l'on utilise comme agent de dispersion de la poly-N-vinyl-2- pyrrolidone.

5. Procédé de préparation de matériaux d'enduction pour matériaux contenant du collagène, caractérisé en ce que l'on mélange avec agitation
   a) 1 - 10% en poids d'aldéhyde,
   b) 10 -40% en poids de monomère soluble dans l'eau avec de l'hydrogène actif,
   c) 1 à 50% en poids de monomère insoluble dans l'eau avec deux ou plusieurs liaisons doubles polymérisables,
   d) 0,01 - 2,5% en poids de photo-initiateur,
   e) 15 à 70% en poids d'eau,
   f) 0,2 à 10% en poids de solubilisant et/ou de dispersant, et éventuellement
   g) 0 à 5% en poids d'additif connu en soi.

**Revendication pour l'Etat contractant suivant : ES**

1. Procédé de préparation d'un agent d'enduction pour matériaux contenant du collagène, caractérisé en ce que l'on mélange avec agitation :
   a) 1 - 10% en poids d'aldéhyde,
   b) 10 - 40% en poids de monomère soluble dans l'eau avec de l'hydrogène actif,
   c) 1 à 50% en poids de monomère insoluble dans l'eau avec deux ou plusieurs liaisons doubles polymérisables,
   d) 0,01 - 2,5% en poids de photo-initiateur,
   e) 15 à 70% en poids d'eau,
   f) 0,2 à 10% en poids de solubilisant et/ou de dispersant, et éventuellement
   g) 0 à 5% en poids d'additif connu en soi.